# FASCICULE DE BREVET EUROPEEN

(11) **EP 0 820 972 B1**
(45) Date de publication et mention de la délivrance du brevet: **20.06.2001**
(21) Numéro de dépôt: 97401438.3
(22) Date de dépôt: 20.06.1997
(51) Int. Cl.: C07C 9/00, C07C 7/13, C10G 25/03

(54) **Procédé de séparation isoalcanes/n-alcanes par adsorption en phase gazeuse utilisant une modulation de pression et quatre adsorbeurs**
Verfahren zur Isoalkan/n-Alkan-Trennung durch Adsorption in der Gasphase in dem Druckmodulierung und vier Adsorber verwendet werden
Process for the isoalkane/n-alkane separation by adsorption in the gas phase using pressure modulation and four adsorbers

(30) Priorité: 26.07.1996 FR 9609551
(43) Date de publication de la demande: 28.01.1998
(73) Titulaire: INSTITUT FRANCAIS DU PETROLE, 92852 Rueil-Malmaison Cedex (FR)
(72) Inventeur: Ambrosino, Jean-Louis, 69360 Ternay (FR); Chansolme, Alain, 78400 Chatou (FR); Wiss-Henrard, Valérie, 78220 Viroflay (FR)

(56) Documents cités:
- EP-A- 0 006 665
- EP-A- 0 103 070
- FR-A- 2 325 624

## Description

La présente invention est relative à un procédé d'adsorption et plus particulièrement à un procédé d'adsorption en phase vapeur mettant en jeu des mécanismes de séparation basés sur des phénomènes d'exclusion ou de différence d'adsorption.

Elle concerne plus particulièrement un procédé de séparation utilisant de façon judicieuse les enchaînements de séquences dans un PSA afin de diminuer le rôle pénalisant de la zone de transfert de matière.

La présente invention s'applique à la séparation par adsorption sur tamis zéolithique d'un mélange de paraffines linéaires d'un mélange de paraffines non linéaires et plus particulièrement la séparation d'iso-paraffines et de paraffines normales à partir d'une charge d'hydrocarbures en contenant. Plus particulièrement encore, la charge d'hydrocarbures à traiter provient d'une opération d'isomérisation d'une coupe d'hydrocarbures en C5/C6.

Le PSA (abréviation de l'anglais "Pressure Swing Adsorption") est un procédé d'adsorption dans lequel un mélange gazeux est mis en contact avec un lit fixe d'adsorbant à haute pression afin d'éliminer certains constituants dits "adsorbables" du mélange. Même si la désorption peut être réalisée par différents moyens, la caractéristique commune de la famille des PSA est d'effectuer la régénération du lit par dépressurisation et, dans certains cas, par balayage à basse pression.

Ces PSA ont remporté de nombreux succès dans le domaine du gaz naturel, de la séparation des composés de l'air, de la production de solvants et dans différents secteurs du raffinage. Une revue des étapes importantes du développement des PSA a été donnée en 1984 dans un article de l"'AIChE Symposium Series" intitulé "Twenty Five Years Progress in Adiabatic Adsorption Process" par R.T.Cassidy et E.S.Holmes.

Les procédés de séparation par PSA fournissent un moyen économique et efficace pour séparer les constituants d'un gaz contentant au moins deux composés ayant des caractéristiques d'adsorption différentes. Le constituant adsorbable peut être une impureté à éliminer du constituant le moins adsorbable, ce dernier étant alors récupéré comme produit. Ou bien c'est le produit le plus adsorbable qui est désiré et doit être séparé du constituant le moins adsorbable. Par exemple, on peut vouloir éliminer le monoxyde de carbone et les composés légers à partir d'un flux riche en hydrogène pour produire de l'hydrogène à une pureté supérieure à 99 %, utilisable dans un hydrocracking ou tout autre procédé catalytique, dans lequel le catalyseur est sensible aux impuretés. D'un autre coté, ce peut être le produit le plus adsorbable qu'il est utile de récupérer, comme par exemple l'éthylène à partir d'une charge pour obtenir un produit riche en éthylène.

Dans les cas les plus classiques de PSA, une charge multi-composants gazeuse est introduite dans au moins un des lits d'adsorbants à une pression effective élevée pour adsorber au moins un des constituants (la fraction adsorbée), pendant que les autres constituants (la fraction non adsorbée) passent au travers du lit sans être retenus. À un temps pré-défini, le flux de charge vers cet adsorbeur est interrompu et le lit est dépressurisé par mise en oeuvre d'une ou de plusieurs étapes de dépressurisation en co-courant (pour le sens de l'écoulement, on se référera toujours dans la présente description à celui de l'adsorption), jusqu'à atteindre un niveau de pression pré-défini. Cela permet aux composés les moins adsorbables ou à ceux restant dans la zone d'adsorption (volumes interstitiels) d'être évacués sans être mélangés au constituant le plus adsorbable. Le gaz récupéré pendant ces étapes de dépressurisation est en général utilisé pour réaliser les étapes d'égalisation de pression ou de balayage ultérieures. Dans l'art antérieur et dans la présente description, le vocable "égalisation de pression" est utilisé pour décrire la connexion d'un adsorbeur haute pression avec un adsorbeur basse pression, réalisée jusqu'à ce que soit atteint le même niveau de pression dans les deux adsorbeurs. Le lit est ensuite dépressurisé à contre-courant et souvent balayé pour désorber le composé de la charge le plus fortement adsorbé et pour évacuer ce gaz du lit avant les étapes de pressurisation puis d'adsorption. De tels procédés sont décrits par exemple dans le brevet US-A-3 430 418 ou dans l'ouvrage plus général de R.T.Yang : "Gas Separation by Adsorption Processes", Boston, Butterworth (1987), dans lequel des cycles basés sur l'utilisation de plusieurs lits sont décrits en détail. En général, et tel que c'est décrit dans les ouvrages mentionnés, les procédés de type PSA sont mis en oeuvre de façon séquentielle et par utilisation alternative de tous les lits d'adsorption.

On connaît la demande EP-A-0 103 070 qui décrit un procédé dans lequel des gaz fortement adsorbés sur des adsorbants (le constituant gazeux recherché), en particulier le méthane ou le CO₂, sont séparés et récupérés à partir de mélanges gazeux qui contiennent en outre essentiellement des gaz peu adsorbés (le constituant gazeux à éliminer), par utilisation d'adsorbants contenant du carbone et suivant une technique PSA (« Pressure Swing Adsorption »). Le procédé peut être décrit comme suit :
Au cours de la phase d'adsorption, on fait passer le mélange de départ à travers un lit d'adsorption qui adsorbe le « gaz recherché » et évacue le « gaz à éliminer » jusqu'au perçage du « gaz recherché » à la sortie du lit. Dans une phase ultérieure de balayage à la pression d'adsorption, l'adsorbeur est balayé par un flux du « gaz recherché » ayant la qualité désirée, au moins vers la fin du balayage ; dans cette phase, la recette initiale est considérée comme « gaz à éliminer » et la recette ultérieure, contenant une proportion plus importante de « gaz recherché », est recyclée vers l'alimentation du procédé PSA.
Dans la phase de désorption, la pression est réduite dans l'adsorbeur et on obtient le « gaz recherché » hautement enrichi ; à partir de celui-ci, on recycle encore une portion pour balayer le PSA et on récupère le reste comme produit.
Après que la désorption est terminée, la pression gazeuse dans l'adsorbeur est à nouveau accrue au moyen d'un mélange gazeux venant du PSA pour la phase d'adsorption suivante.
Quand le mélange gazeux de départ contient 15 % ou moins de CO₂ ou de méthane, la mise sous pression dans l'adsorbeur est réalisée en utilisant le mélange gazeux de départ.

On connaît en outre la demande de brevet français FR-A-2 325 624 qui décrit un procédé de séparation de n-paraffines à partir d'une charge contenant en outre des isoparaffines et des hydrocarbures aromatiques, ledit procédé comprenant l'adsorption sélective sur une zéolithe et la désorption subséquente par un agent désorbant, réalisée en deux étapes. Un premier agent désorbant déplace les hydrocarbures aromatiques de la surface de la zéolithe, tandis que le second agent désorbant déplace les n-paraffines des pores.

On connaît enfin la demande européenne EP-A-0 006 665 qui décrit un procédé de séparation de paraffines linéaires d'un mélange consistant principalement en paraffines linéaires et en paraffines ramifiées, ce procédé comprenant successivement une étape (a) dans laquelle on fait passer la charge de haut en bas à travers un lit de tamis moléculaire ne contenant pratiquement pas de paraffines linéaires et dans lequel les vides contiennent des paraffines ramifiées, jusqu'à ce qu'une quantité désirée de paraffines linéaires ait été adsorbée sur le tamis moléculaire ; une étape (b) dans laquelle on fait passer un mélange de paraffines linéaires et d'hydrogène de haut en bas à travers le lit de tamis moléculaire jusqu'à ce que pratiquement toutes les paraffines ramifiées en soient chassées ; une étape (c) dans laquelle on fait passer de l'hydrogène de bas en haut à travers le lit de tamis moléculaire jusqu'à ce que la majeure partie des paraffines linéaires aient été désorbées et évacuées ; et une étape (d) dans laquelle on fait passer les paraffines ramifiées de bas en haut à travers le lit de tamis moléculaire jusqu'à ce que pratiquement tout l'hydrogène en soit chassé.

Les sélectivités qui sont employées dans les procédés PSA s'appuient sur trois mécanismes de base : la sélectivité diffusionnelle, la sélectivité de forme (ou exclusion) et la sélectivité énergétique.

Dans le cas de la sélectivité diffusionnelle, la force motrice pour la séparation est la différence de vitesse d'adsorption, de désorption et de diffusion des différents composés dans le mélange de gaz à séparer.

Dans le cas de sélectivité de forme (ou géométrique), la séparation se fait sur la base de la taille des différentes molécules à séparer par rapport à celle des micro-pores de l'adsorbant. Les molécules les plus grosses ne pénètrent pas la porosité de l'adsorbant, tandis que les plus petites s'adsorbent : on parle d'effet de tamisage et la dimension des pores de l'adsorbant détermine quels sont les composés qui s'adsorbent et ceux qui ne s'adsorbent pas.

Pour ce qui concerne la sélectivité énergétique, c'est l'affinité de l'adsorbant ou les forces relatives d'adsorption pour l'un ou l'autre des composés qui contrôlent la séparation. Le composé le moins fortement adsorbé devient la fraction non adsorbable et le plus fortement adsorbé devient la fraction adsorbable.

Dans le cas des procédés basés sur des sélectivités diffusionnelles ou géométriques, la vitesse de transfert de matière a une importance sur l'efficacité et la taille des lits. Compte tenu de ce phénomène, il existe un profil de concentration de l'adsorbat dans le lit au fur et à mesure que le front d'adsorption progresse dans celui-ci. Le terme "zone de transfert de matière" (abrégé en anglais "MTZ") est connu de l'homme de l'art et se réfère à la région qui contient ce profil. Dans cette région, la concentration en adsorbat est nulle à une extrémité (vers la sortie du lit) et égale à celle dans la charge à l'autre (vers l'entrée de la charge). Dans le but de produire un flux à haute pression et haute pureté, le cycle d'adsorption du PSA est déterminé de telle sorte que cette zone de transfert de matière ne sorte pas du lit. Il en résulte que l'adsorbant dans la MTZ n'est pas saturé à sa capacité d'équilibre et qu'il y a donc une perte d'efficacité du système. De nombreux travaux ont porté sur la réduction de la zone de transfert de matière par action sur les paramètres hydrodynamiques du système. De la réduction de cette zone de transfert de matière et donc de la partie inutilisée du lit, résulte une diminution de l'inventaire sur l'adsorbant et donc de la taille des lits.

Un objet de l'invention est de fournir un moyen de diminuer la taille du lit, ou inversement d'augmenter la capacité dynamique de l'adsorbant, dans le cas des procédés classiques de PSA utilisant une sélectivité de type géométrique. Cette augmentation de capacité est réalisée sans (ou à moindre) augmentation d'investissement ou de coûts opératoires.

Il a en effet été découvert que la quantité de tamis à mettre en jeu, pour une séparation donnée, pouvait être diminuée par un arrangement judicieux des séquences. Cette augmentation des performances peut être analysée de différentes façons : soit en termes d'augmentation de capacité dynamique, soit en termes d'augmentation de VVH (vitesse volumique horaire) à performance fixée. Elle est due au fait que, compte tenu de l'arrangement des séquences subies par les différents adsorbeurs, qui seront décrites ci-après, le lit en fin d'adsorption est complètement saturé par le (ou les) composé(s) adsorbable(s), c'est-à-dire que la zone de transfert de matière est sortie de l'adsorbeur avant que celui-ci soit régénéré.

Le procédé de la présente invention peut s'appliquer à l'obtention d'un mélange riche en iso-paraffines conduisant à un produit à haut indice d'octane, à partir d'un mélange contenant également des paraffines normales.

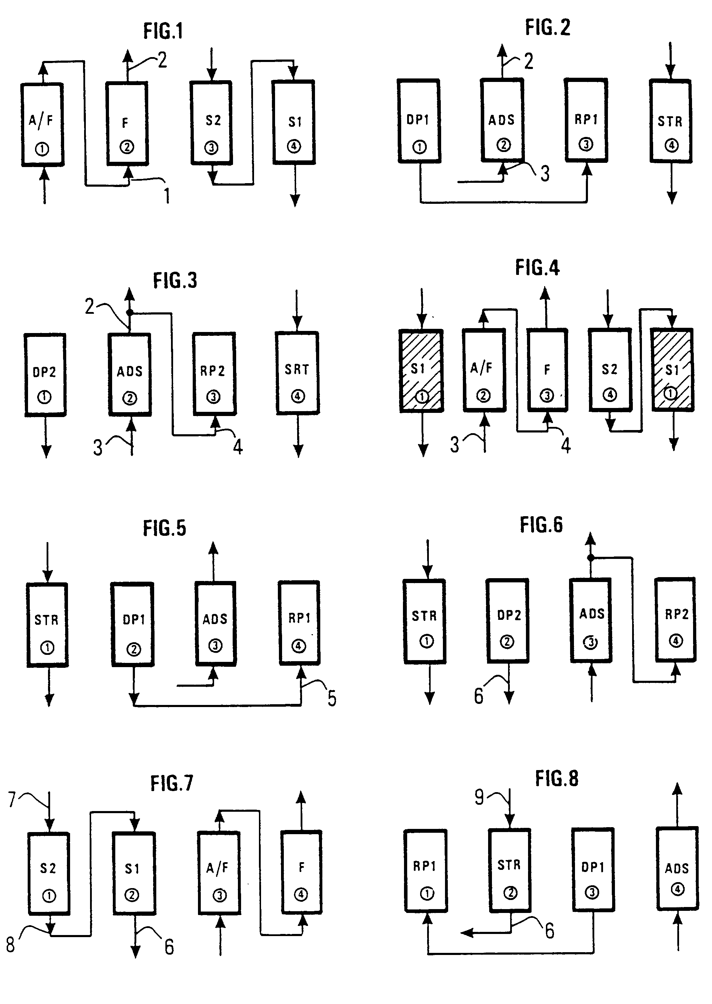

Le procédé de l'invention est décrit ci-après en liaison avec le tableau 1 qui suit et les figures 1 à 12, qui illustrent respectivement les séquences de 1 à 12.

L'invention fournit donc un procédé de séparation de n-paraffines et d'iso-paraffines à partir d'un mélange en contenant, par adsorption en phase gazeuse des n-paraffines, ce procédé utilisant quatre adsorbeurs, qui seront numérotés dans la suite de 1 à 4, ayant substantiellement la même taille et fonctionnant selon au moins un cycle d'étapes qui sera décrit ci-après en référence à l'adsorbeur 2 des figures 1 à 12. Le mélange traité provient plus particulièrement d'une isomérisation d'hydrocarbures en C5/C6.

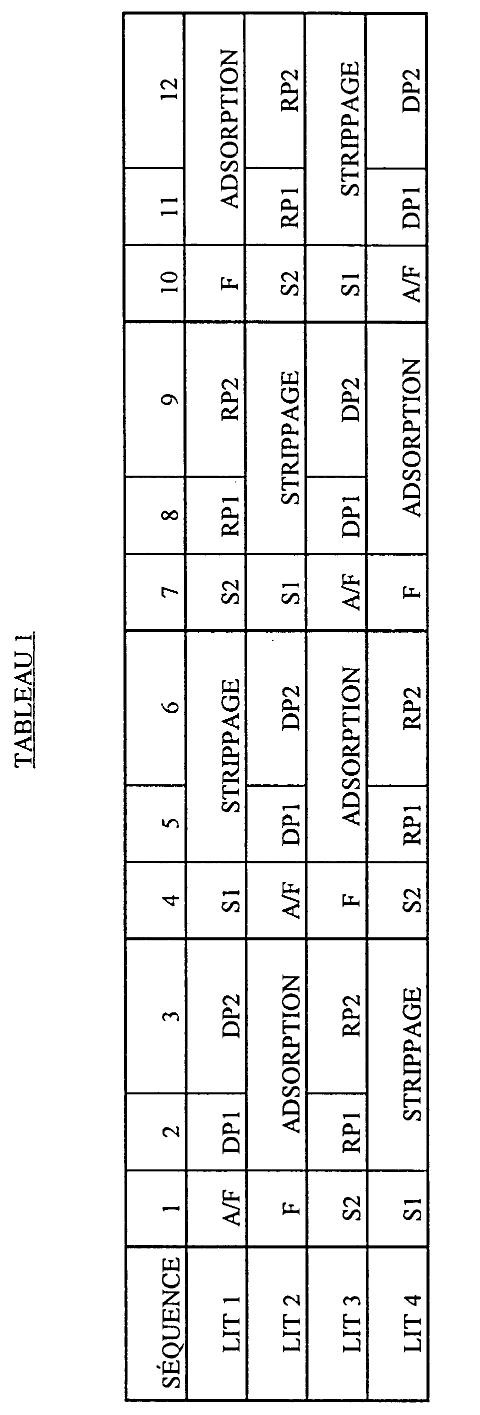

Dans la description qui suit, on utilisera indifféremment les termes "lit" ou "adsorbeur" pour désigner les lits d'adsorption, même lorsque le lit considéré n'est pas en phase d'adsorption.

Le cycle caractéristique du procédé de l'invention peut être défini comme suit.
a) Séquence 1 : Dans une étape d'adsorption (F), on fait circuler en courant ascendant sur l'adsorbeur 2 une charge constituée de l'effluent de l'adsorbeur qui le précède (adsorbeur 1). Pour cela, on connecte en série la sortie de l'adsorbeur 1, en phase d'adsorption, avec le bas de l'adsorbeur 2, par la ligne 1. Le fluide circule en courant ascendant dans les deux adsorbeurs. Le produit riche en iso-paraffines est récupéré en partie haute de l'adsorbeur 2 par la ligne 2.
b) Séquence 2 : Dans une étape d'adsorption (ADS), on injecte directement la charge en courant ascendant au bas de l'adsorbeur 2 par la ligne 3. Le produit riche en isoparaffines est récupéré en partie haute de l'adsorbeur 2 par la ligne 2.
c) Séquence 3 : On poursuit l'opération d'adsorption (ADS) et, par la ligne 4, on prélève, sur la ligne 2, une partie du fluide de sortie riche en isoparaffines pour réaliser la seconde étape de pressurisation RP2 de l'adsorbeur suivant 3 en courant ascendant.
d) Séquence 4 : On continue l'étape d'adsorption (A/F) et l'on connecte, par la ligne 4, l'extrémité haute de l'adsorbeur 2 au bas de l'adsorbeur suivant (adsorbeur 3), précédemment en phase de seconde pressurisation. Au cours de cette étape, l'adsorbeur 2 recevant la charge par la ligne 3 se trouve saturé en n-paraffines et la zone de transfert de matière se déplace vers l'adsorbeur suivant 3, connecté en série.
e) Séquence 5 : On effectue une première dépressurisation (DP1) en connectant par le bas, par la ligne 5, l'adsorbeur 2 fonctionnant à haute pression avec l'adsorbeur 4 ayant terminé son étape de balayage à une pression plus faible. On obtient ainsi un équilibrage de la pression dans les adsorbeurs 2 et 4. L'adsorbant dans l'adsorbeur 2 est donc totalement saturé à l'équilibre de la charge.
f) Séquence 6: On réalise une seconde dépressurisation (DP2), l'extrémité supérieure de l'adsorbeur2 étant fermée; si la charge traitée par le procédé provient d'une unité d'isomérisation, par exemple d'hydrocarbures en C5/C6, on peut connecter le bas de l'adsorbeur 2, par la ligne 6, avec le circuit de recyclage vers l'isomérisation, maintenu à basse pression.
g) Séquence 7 : Dans une première phase de balayage (S1), on relie le bas de l'adsorbeur précédent 1 au haut de l'adsorbeur 2 par la ligne 8, on injecte le désorbant par la ligne 7 en tête de l'adsorbeur 1 en fin de balayage (S2); l'effluent qui en sort est alors relativement pauvre en fraction adsorbable. La phase de balayage (S1) a lieu en général à une pression inférieure à 5 bars et de préférence inférieure à 3 bars absolus et s'effectue à contre-courant par rapport à l'étape d'adsorption. Le produit sortant par la ligne 6 de l'adsorbeur 2 consiste essentiellement une fraction riche en n-paraffines. Si la charge traitée par le procédé provient d'une unité d'isomérisation, par exemple d'hydrocarbures en C5/C6, on peut recycler cet effluent vers l'unité d'isomérisation.
h) Séquences 8 et 9 : Dans l'étape principale de balayage (STR), on alimente uniquement l'adsorbeur 2 par le désorbant en écoulement descendant, par la ligne 9. Dans ce cas également, on peut recycler l'effluent sortant par la ligne 6 vers l'unité d'isomérisation.
i) Séquence 10 : Dans l'étape de finition du balayage (S2), on continue à alimenter en désorbant l'adsorbeur 2, par la ligne 9, mais on connecte la sortie de l'adsorbeur 2 à l'adsorbeur suivant 3, qui amorce la phase de désorption (S1).
j) Séquence 11 : On réalise une pressurisation (RP1) de la pression basse à une pression intermédiaire en connectant les adsorbeurs et 4 par le bas par la ligne 11.
k) Séquence 12 : Enfin, on réalise une seconde pressurisation (RP2) jusqu'à la pression d'adsorption au moyen d'un flux provenant par la ligne 12 en partie de l'effluent de l'adsorbeur précédent 1, qui fonctionne dans une étape d'adsorption (ADS). Le fluide est introduit en partie basse de l'adsorbeur 2 pour éviter de polluer le lit si ce fluide n'est pas parfaitement exempt de produits adsorbables.

En résumé, les n-paraffines sont adsorbées à haute pression (étapes F et ADS) et désorbées à pression plus faible par mise en jeu d'un effet d'abaissement de la pression, combiné à un effet d'élimination par "balayage" à l'aide d'un flux gazeux riche en iso-paraffines. Le désorbant peut contenir une proportion de n-paraffines comprise entre 0 et 20 %.

Le lit adsorbant est constitué en général d'un tamis moléculaire à base de zéolithe capable d'adsorber sélectivement les n-paraffines et ayant un diamètre apparent de pores voisin de 5 Angströms. La zéolithe 5A convient pour cet usage : son diamètre de pores est proche de 5 Å et sa capacité d'adsorption pour les n-paraffines est importante. On peut cependant employer d'autres adsorbants tels que la chabazite ou l'érionite.

Les conditions opératoires préférées sont des températures de 100 à 400°C et plus, préférentiellement de 200 à 300°C, et une pression d'adsorption de 5 à 40 bars et plus, préférentiellement de 15 à 25 bars. Le cycle d'adsorption dure en général de 2 à 15 minutes et préférentiellement de 4 à 6 minutes.

L'exemple 1 qui suit, non limitatif, illustre l'invention. L'exemple 2 est donné à titre comparatif.

### Exemple 1

Le procédé de l'invention est mis en oeuvre dans une unité comportant quatre adsorbeurs identiques effectuant le cycle précédemment décrit.

Les adsorbeurs sont des cylindres de 0,053 m de diamètre interne et de 4,77 m de hauteur, contenant chacun 8,05 kg de tamis SA. La charge et le désorbant sont introduits à une température maintenue à 215°C.

La charge est constituée d'un naphta léger et provient de l'isomérisation d'une coupe pétrolière en C5/C6 ayant la composition massique suivante :

| Constituant | % masse |
|---|---|
| Isobutane (iC4) | 1,39 |
| Normal butane (nC4) | 1,02 |
| Isopentane (iC5) | 27,99 |
| Normal pentane (nC5) | 11,2 |
| 2,2-diméthylbutane (22 DMB) | 11,3 |
| 2,3-diméthylbutane (23 DMB) | 4,8 |
| 2-méthylpentane (2 MC5) | 14,6 |
| 3-méthylpentane (3 MC5) | 8,7 |
| Normal hexane (nC6) | 6,1 |
| Cyclopentane (CC5) | 2,0 |
| Méthylcyclopentane (MC5) | 5,7 |
| Cyclohexane (CC6) | 5,2 |
| TOTAL | 100,00 |

La charge et le désorbant alimentent l'unité de séparation sous contrôle de débit et les effluents sont recueillis sous contrôle de pression.

Le débit de charge est de 19,65 kg/h, son indice d'octane (RON) est de 81,6.

Le débit de désorbant est égal à 6,74 kg/h ; sa composition massique figure ci-après :

| Constituant | % masse |
|---|---|
| Isobutane (iC4) | 4,25 |
| Normal butane (nC4) | 3,39 |
| Isopentane (iC5) | 92,26 |
| Normal pentane (nC5) | 0,1 |
| TOTAL | 100,00 |

La pression d'adsorption est comprise entre 17,7 et 17,1 bars absolus, tandis que la pression de désorption est de 2,6 bars absolus.

La durée en secondes des différentes séquences du cycle est indiquée pour un quart du cycle dans le tableau suivant :

| Séquence | 1 | 2 | 3 |
|---|---|---|---|
| lit 1 | A/F | DP1 | DP2 |
| lit 2 | F | ADS | ADS |
| lit 3 | S2 | RP1 | RP2 |
| lit 4 | S1 | STR | STR |
| durée (en s) | 60 | 40 | 100 |

La durée globale de la phase d'adsorption pour un même lit est égale à la somme des séquences F et ADS, soit ici 200 secondes. Cette somme correspond également à la durée totale de désorption pour un adsorbeur donné, somme des séquences STR et S1.

On peut noter que, du fait que le désorbant alimente toujours un adsorbeur, il n'est pas nécessaire de prévoir une vanne de "by-pass" de l'unité (comme dans le cas de l'exemple comparatif 2 présenté ci-après).

Les bilans de l'unité sont réalisés à l'état cyclique convergé, c'est-à-dire tel que deux bilans successifs conduisent au même résultat.

L'effluent riche en iso-paraffines est désigné par 1"'IPSORBAT". Il a une composition massique moyenne cumulée sur une heure de fonctionnement donnée ci-dessous

| Constituant | % masse |
|---|---|
| Isobutane (iC4) | 1,32 |
| Normal butane (nC4) | 1,81 |
| Isopentane (iC5) | 36,49 |
| Normal pentane (nC5) | 1,13 |
| 2,2-diméthylbutane (22 DMB) | 12,67 |
| 2,3-diméthylbutane (23 DMB) | 5,38 |
| 2-méthylpentane (2 MC5) | 16,38 |
| 3-méthylpentane (3 MC5) | 9,77 |
| Normal hexane (nC6) | 0,59 |
| Cyclopentane (CC5) | 2,24 |
| Méthylcyclopentane (MC5) | 6,39 |
| Cyclohexane (CC6) | 5,83 |
| TOTAL | 100,00 |

Le débit d"'IPSORBAT" est de 14,17 kg/h ; ce produit a un indice d'octane (RON) de 88,0.

### Exemple 2 (comparatif)

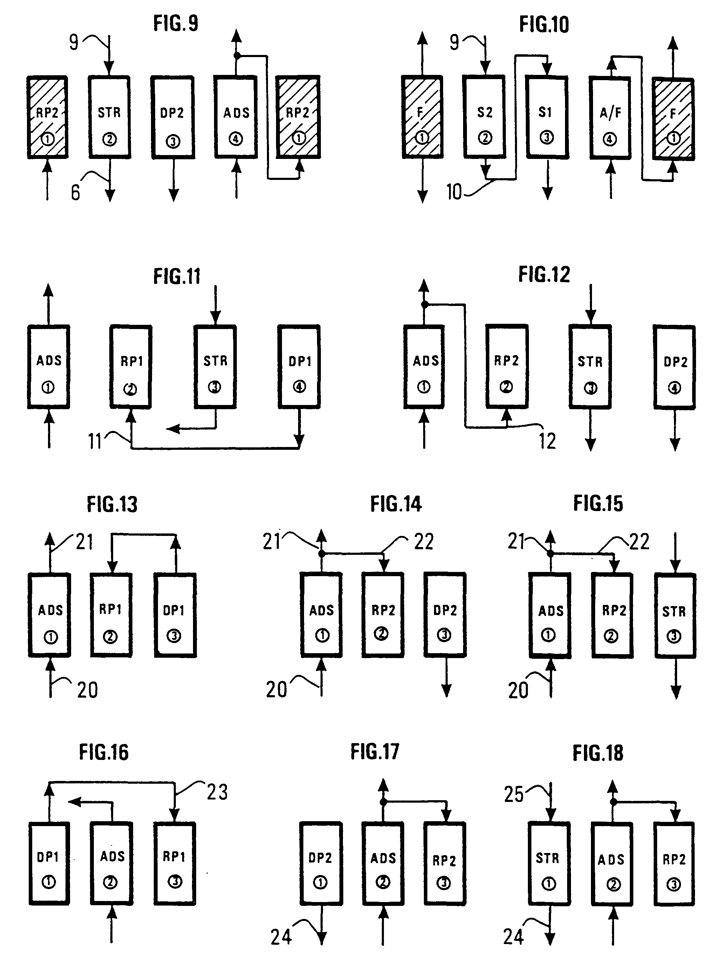

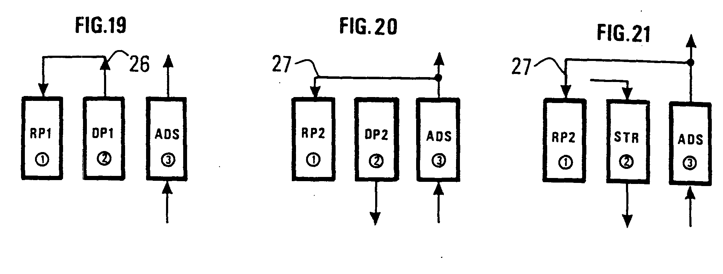

La différence avec l'exemple précédent réside dans l'emploi de trois adsorbeurs au lieu de quatre et l'unité fonctionne selon un cycle connu, décrit ci-après en liaison avec le tableau 2 et les figures 13 à 21, qui illustrent respectivement les séquences de 1 à 9.

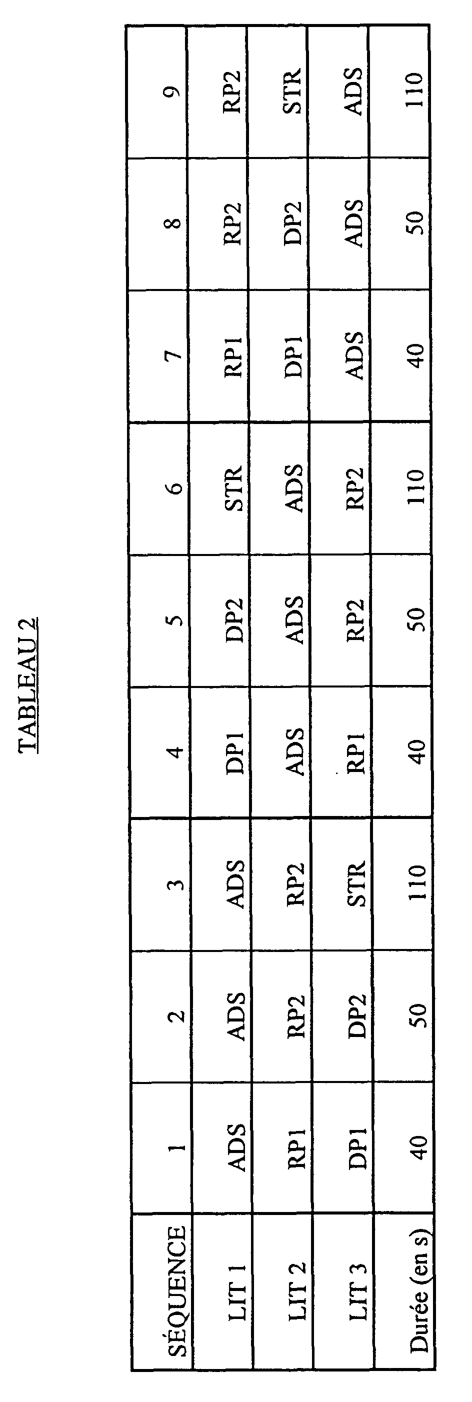

Le cycle suivi par chaque adsorbeur se déroule selon neuf séquences. Ces séquences sont identiques pour les trois adsorbeurs, mais elles sont décalées dans le temps d'une durée égale au tiers de la durée globale du cycle. Par exemple, le lit 1 effectuera les séquences suivantes :
- une séquence 1 dans laquelle on réalise une étape d'adsorption (ADS) à haute pression en introduisant la charge en bas du lit 1 par la ligne 20 et en la faisant passer en courant ascendant à travers ledit lit, le produit sortant en tête par la ligne 21 ;
- deux séquences 2 et 3 dans lesquelles on poursuit l'étape d'adsorption (ADS) en courant ascendant et on récupère sur l'effluent sortant de l'adsorbeur 1 par la ligne 21 une partie que l'on envoie par la ligne 22 vers l'adsorbeur suivant 2, pour en effectuer la seconde pressurisation (RP2) ;
- une séquence 4 dans laquelle on réalise une étape d'équilibrage de pression (DP1) en connectant par leurs extrémités hautes par la ligne 23 l'adsorbeur 1 avec l'adsorbeur 3 en fin de balayage (STR) pour en amorcer la repressurisation ;
- une séquence 5 dans laquelle on réalise une seconde dépressurisation (DP2) en connectant la partie basse de l'adsorbeur 1 avec le circuit de recyclage vers l'isomérisation par la ligne 24 ;
- une séquence 6 dans laquelle on réalise une étape de balayage (STR) en introduisant le désorbant en courant descendant sur le lit 1 par la ligne 25, l'effluent de sortie contenant les n-paraffines adsorbées étant recyclée par la ligne 24 vers l'unité d'isomérisation ;
- une séquence 7 dans laquelle on réalise une étape de pressurisation (RP1) par mise en connexion du lit 1 avec le lit 2 par la ligne 26 ; et
- deux séquences 8 et 9 dans lesquelles on réalise une étape de seconde pressurisation (RP2) en reliant par la ligne 27 le lit 1 avec le lit 3, qui fonctionne alors dans une étape d'adsorption (ADS).

Chaque adsorbeur à un diamètre interne 0,053 m et une hauteur de 6,36 m et contient 10,7 kg de tamis 5A. On utilise donc la même masse totale de tamis. Les compositions ainsi que les débits de charge et de désorbant sont les mêmes que dans l'exemple 1 selon l'invention. Il en est de même pour leurs températures d'entrée.

Les pressions d'adsorption sont comprises entre 17,7 et 17,4 bars absolus et la pression de désorption est de 2,6 bars absolus.

La durée des différentes séquences du cycle est indiquée dans le tableau 2, un cycle s'effectuant en 600 secondes. On notera que le temps des séquences d'adsorption et d'équilibrage de pression (DP1/RP1) sont les mêmes que dans l'exemple 1 selon l'invention. Cependant, il est nécessaire de prévoir un "by-pass" de l'unité pendant les séquences où le désorbant n'est pas utilisé (DP1/DP2).

L'effluent recueilli dans le procédé, riche en iso-paraffines a une composition massique moyenne cumulée sur une heure de fonctionnement donnée ci-dessous:

| Constituant | % masse |
|---|---|
| Isobutane (iC4) | 1,78 |
| Normal butane (nC4) | 1,31 |
| Isopentane (iC5) | 36,08 |
| Normal pentane (nC5) | 2,45 |
| 2,2- diméthylbutane (22 DMB) | 12,33 |
| 2,3-diméthylbutane (23 DMB) | 5,24 |
| 2-méthylpentane (2 MC5) | 15,93 |
| 3-méthylpentane (3 MC5) | 9,50 |
| Normal hexane (nC6) | 1,31 |
| Cyclopentane (CC5) | 2,18 |
| Méthylcyclopentane (MC5) | 6,22 |
| Cyclohexane (CC6) | 5,67 |
| TOTAL | 100,00 |

Le débit d'"IPSORBAT" est de 13,77 kg/h et a un indice d'octane (RON) de 87,3.

La comparaison des résultats de l'exemple 1 selon l'invention et de l'exemple comparatif2 montre que le gain d'octane est plus important si l'on procède selon l'invention, puisque, dans ce dernier cas, on obtient un indice d'octane de 88,0.

## Revendications

1. Procédé de séparation par adsorption en phase gazeuse de n-paraffines et d'iso-paraffines à partir d'une charge d'hydrocarbures provenant d'une étape d'isomérisation, ledit procédé étant caractérisé en ce qu'il utilise quatre adsorbeurs, numérotés de 1 à 4, ayant substantiellement la même taille, contenant le même adsorbant et fonctionnant chacun selon au moins un cycle d'étapes qui, décrit en référence à l'adsorbeur 2, tel que représenté, ainsi que les adsorbeurs 1, 3 et 4, sur les figures 1 à 12, comprend :
a) une séquence 1 dans laquelle on réalise une étape d'adsorption (F), en faisant circuler en courant ascendant sur l'adsorbeur 2 une charge constituée de l'effluent de l'adsorbeur 1 qui le précède, en connectant en série la sortie de l'adsorbeur 1, en phase d'adsorption, avec le bas de l'adsorbeur 2 par une ligne 1 et on récupère un produit riche en iso-paraffines en partie haute de l'adsorbeur 2 par une ligne 2 ;
b) une séquence 2, dans laquelle on réalise une étape d'adsorption (ADS), en injectant directement la charge en courant ascendant au bas de l'adsorbeur 2 par une ligne 3 et on récupère un produit riche en iso-paraffines en partie haute de l'adsorbeur 2 par la ligne 2 ;
c) une séquence 3 dans laquelle on poursuit l'opération d'adsorption (ADS) et, par une ligne 4, on prélève sur la ligne 2 une partie du fluide de sortie riche en iso-paraffines pour réaliser la seconde étape de pressurisation RP2 de l'adsorbeur suivant 3, en courant ascendant ;
d) une séquence 4 dans laquelle on continue l'étape d'adsorption (A/F) en connectant, par une ligne 4, l'extrémité haute de l'adsorbeur 2 au bas de l'adsorbeur suivant 3, précédemment en phase de seconde pressurisation, l'adsorbeur 2 recevant la charge par la ligne 3 étant alors saturé en n-paraffines et la zone de transfert de matière se déplaçant vers l'adsorbeur suivant 3, connecté en série ;
e) une séquence 5 dans laquelle on effectue une première dépressurisation (DP1) en connectant par le bas, par une ligne 5, l'adsorbeur 2 fonctionnant à haute pression avec l'adsorbeur 4 ayant terminé son étape de balayage à une pression plus faible, obtenant ainsi un équilibrage de la pression dans les adsorbeurs 2 et 4, l'adsorbant dans l'adsorbeur 2 étant alors totalement saturé à l'équilibre de la charge ;
f) une séquence 6 dans laquelle on réalise une seconde dépressurisation (DP2), l'extrémité supérieure de l'adsorbeur 2 étant fermée, l'effluent de cette séquence étant évacué par le bas de l'adsorbeur 2 par une ligne 6 ;
g) une séquence 7 dans laquelle on réalise une première phase de balayage (Si) en connectant en série le bas de l'adsorbeur précédent 1 au haut de l'adsorbeur 2 par une ligne 8, en injectant le désorbant par une ligne 7 en tête de l'adsorbeur 1 en fin de balayage (S2), l'effluent qui en sort étant alors relativement pauvre en fraction adsorbable, le produit sortant par la ligne 6 de l'adsorbeur 2 consistant essentiellement en une fraction riche en n-paraffines ;
h) deux séquences 8 et 9 dans lesquelles on réalise l'étape principale de balayage (STR) en alimentant uniquement l'adsorbeur 2 par le désorbant en écoulement descendant par une ligne 9 ;
i) une séquence 10 dans laquelle on réalise une étape de finition du balayage (S2) en continuant à alimenter en désorbant l'adsorbeur 2 par la line 9 et en connectant par une ligne 10 la sortie dudit adsorbeur 2 à l'adsorbeur suivant 3, qui amorce la phase de désorption (S1);
j) une séquence 11 dans laquelle on réalise une pressurisation (RP1) de la pression basse à une pression intermédiaire en connectant les adsorbeurs 2 et 4 par le bas par une ligne 11 ; et
k) une séquence 12 dans laquelle on réalise une seconde pressurisation (RP2) jusqu'à la pression d'adsorption au moyen d'un flux provenant par une ligne 12 en partie de l'effluent de l'adsorbeur précédent 1, qui fonctionne dans une étape d'adsorption (ADS), le fluide étant introduit en partie basse de l'adsorbeur 2 ;
étant entendu que :
- l'adsorbeur 3 amorce sa séquence 1 lorsque l'adsorbeur 2 amorce sa séquence 4 ;
- l'adsorbeur 4 amorce sa séquence 1 lorsque l'adsorbeur 2 amorce sa séquence 7 ; et
- l'adsorbeur 1 amorce sa séquence 1 lorsque l'adsorbeur 2 amorce sa séquence 10.

2. Procédé selon la revendication 1 caractérisé en ce que, dans les séquences 7 à 10, le balayage est effectué à l'aide d'un flux gazeux riche en iso-paraffines.

3. Procédé selon la revendication 2 caractérisé en ce que le désorbant contient une proportion de n-paraffines comprise entre 0 et 20 %.

4. Procédé selon l'une des revendications 1 à 3 caractérisé en ce que le lit adsorbant est constitué d'un tamis moléculaire à base de zéolithe capable d'adsorber sélectivement les n-paraffines et ayant un diamètre apparent de pores voisin de 5 Angströms.

5. Procédé selon la revendication 4 caractérisé en ce que le lit adsorbant est constitué de zéolithe 5A, de chabazite ou d'érionite.

6. Procédé selon l'une des revendications 1 à 5 caractérisé en ce que l'on opère à des températures de 100 à 400°C et à une pression d'adsorption de 5 à 40 bars, le cycle d'adsorption durant de 2 à 15 minutes.

7. Procédé selon la revendication 6 caractérisé en ce que l'on opère à des températures de 200 à 300°C et à une pression d'adsorption de 15 à 25 bars, le cycle d'adsorption durant de 4 à 6 minutes.

8. Procédé selon l'une des revendications 1 à 7 caractérisé en ce que, dans l'étape (g), la phase de balayage est effectuée à une pression inférieure à 5 bars absolus.

9. Procédé selon l'une des revendications 1 à 7 caractérisé en ce que, dans l'étape (g), la phase de balayage est effectuée à une pression inférieure à 3 bars absolus.

10. Procédé selon l'une des revendications 1 à 9 caractérisé en ce que la charge traitée provient d'une unité d'isomérisation des C5/C6.

11. Procédé selon la revendication 10 caractérisé en ce que l'effluent de désorption de l'adsorbeur 2 est recyclé vers l'unité d'isomérisation des C5/C6, par connexion du bas de l'adsorbeur 2 avec le circuit de recyclage vers l'isomérisation, maintenu à basse pression, dans les séquences 6, 7, 8 et 9.

## Claims

1. A process for separating n-paraffins and isoparaffins by gas phase adsorption from a hydrocarbon feed originating from an isomerisation step, said process being characterized in that it uses four adsorbers, numbered 1 to 4, having substantially the same size, containing the same adsorbent and each operating according to at least one cycle of steps which, described with reference to adsorber 2, as shown, as well as adsorbers 1, 3 and 4, on figures 1 to 12, comprises:
a) a sequence 1 in which an adsorption step (F) is carried out, by circulating a feed constituted by the effluent from preceding adsorber 1 through adsorber 2 as an upflow, by connecting the outlet from adsorber 1, in its adsorption phase, in series with the bottom of adsorber 2 through a line 1 and an isoparaffin-rich product is recovered at the higher part of adsorber 2 through a line 2;
b) a sequence 2, in which an adsorption step (ADS) is carried out, by directly injecting the feed as an upflow into the bottom of adsorber 2 through a line 3 and recovering a product which is rich in isoparaffins from the upper portion of adsorber 2 through line 2;
c) a sequence 3 in which the adsorption operation (ADS) is continued and, through a line 4, a portion of an isoparaffin-rich outlet fluid is extracted on line 2 to carry out a second pressurisation step RP2 of the following adsorber 3, in upflow mode;
d) a sequence 4 in which the adsorption step (A/F) is continued by connecting through line 4 the upper extremity of adsorber 2 to the bottom of the following adsorber 3, previously in the second pressurisation phase, adsorber 2 receiving the feed through line 3 thus being saturated with n-paraffins and the matter transfer zone being displaced towards the following adsorber 3, connected in series;
e) a sequence 5 in which a first depressurisation step (DP1) is carried out by connecting by a line 5 the bottom of adsorber 2 operating at high pressure with the bottom of adsorber 4, which has finished its stripping step and is at a lower pressure, thus equilibrating the pressure in adsorbers 2 and 4, the adsorbent in adsorber 2 being thus totally saturated at the feeding equilibrium;
f) a sequence 6 in which a second depressurisation step (DP2) is carried out, the upper extremity of adsorber 2 being closed, the effluent from this sequence being evacuated at the bottom of adsorber 2 through a line 6;
g) a sequence 7 in which in which a first stripping phase (S1) is carried out by connecting the bottom of the preceding adsorber 1 in series with the top of adsorber 2 through a line 8, by injecting through a line 7 desorbent into the head of adsorber 1 which is at the end of the stripping step (S2), the effluent which leaves therefrom then being relatively depleted in the adsorbable fraction, the product leaving adsorber 2 through line 6 essentially consisting of a fraction which is rich in n-paraffins;
h) two sequences 8 and 9 in which the principal stripping step (STR) is carried out by supplying adsorber 2 alone with a downflow of desorbent through a line 9;
i) a sequence 10 in which a stripping finishing step (S2) is carried out by continuing to supply adsorber 2 through line 9 with desorbent and by connecting the outlet from said adsorber 2 through a line 10 to the following adsorber 3, which is beginning its desorption phase (S1);
j) a sequence 11 in which pressurisation (RP1) is carried out from the low pressure to an intermediate pressure by connecting adsorbers 2 and 4 at the bottom through a line 11; and
k) a sequence 12 in which a second pressurisation (RP2) is carried out to the adsorption pressure by means of a stream which originates through a line 12 from part of the effluent from the preceding adsorber 1, which operates in an adsorption step (ADS), the fluid being introduced to the lower portion of adsorber 2,
provided that:
- adsorber 3 begins its sequence 1 when adsorber 2 begins its sequence 4;
- adsorber 4 begins its sequence 1 when adsorber 2 begins its sequence 7;
- adsorber 1 begins its sequence 1 when adsorber 2 begins its sequence 10.

2. A process according to claim 1, characterized in that in sequences 7 to 10, stripping is carried out using a stream of isoparaffin-rich gas.

3. A process according to claim 2, characterized in that the proportion of n-paraffins contained in the desorbent is in the range 0 to 20%.

4. A process according to any one of claims 1 to 3, characterized in that the adsorbent bed is constituted by a molecular sieve based on zeolite which is capable of selectively adsorbing n-paraffins and with an apparent pore diameter which is approximately 5 Angströms.

5. A process according to claim 4, characterized in that the adsorbent bed is constituted by 5A zeolite, chabazite or erionite.

6. A process according to any one of claims 1 to 5, characterized in that it is carried out at temperatures of 100°C to 400°C and at an adsorption pressure of 5 to 40 bars, the adsorption cycle having a duration of 2 to 15 minutes.

7. A process according to claim 6, characterized in that it is carried out at temperatures of 200°C to 300°C and at an adsorption pressure of 15 to 25 bars, the adsorption cycle having a duration of 4 to 6 minutes.

8. A process according to any one of claims 1 to 7, characterized in that in step (g), the stripping phase is carried out at a pressure of less than 5 bars absolute.

9. A process according to any one of claims 1 to 7, characterized in that, in step (g), the stripping phase is carried out at a pressure of less than 3 bars absolute.

10. A process according to any one of claims 1 to 9, characterized in that the feed which is treated originates from a C5/C6 isomerisation unit.

11. A process according to claim 10, characterized in that in sequences 6, 7, 8 and 9, the desorption effluent from adsorber 2 is recycled to the C5/C6 isomerisation unit by connecting the bottom of adsorber 2 with the circuit which recycles to the isomerisation unit, kept at low pressure.

## Patentansprüche

1. Verfahren zur Trennung, in gasförmiger Phase, durch Adsorption von n-Paraffinen und Isoparaffinen aus einer aus einer Isomerisierungsstufe stammenden Kohlenwasserstoffcharge, dadurch gekennzeichnet, dass das Verfahren vier mit 1 bis 4 bezeichnete Adsorber, die im wesentlichen die gleiche Abmessung haben, über das gleiche Adsorptionsmittel verfügen und je gemäß wenigstens einem Zyklus von Stufen arbeiten, verwendet das, beschrieben mit Bezug auf den Adsorber 2, wie dargestellt, so wie die Adsorber 1, 3 und 4 mit Bezug auf die Figuren 1 bis 12, umfasst:
a) eine Sequenz 1, in der man eine Adsorptionsstufe (F) realisiert, indem man in aufsteigendem Strom am Adsorber 2 eine Charge strömen lässt, die aus dem Abstrom des vorhergehenden Adsorbers 1 gebildet ist, indem man den Ausgang des Adsorbers 1 in der Adsorptionsphase in Reihe mit dem Boden des Adsorbers 2 über eine Leitung 1 verbindet und ein an Isoparaffinen reiches Produkt oben am Adsorber 2 über eine Leitung 2 gewinnt;
b) eine Sequenz 2, in der man eine Adsorptionsstufe (ADS) realisiert, indem man die Charge direkt in aufsteigendem Strom am Boden des Adsorbers 2 über eine Leitung 3 einspritzt und man ein an Isoparaffinen reiches Produkt oben am Adsorber 2 über die Leitung 2 gewinnt;
c) eine Sequenz 3, in der man den Adsorptionsvorgang (ADS) fortsetzt und über eine Leitung 4 an der Leitung 2 einen Teil des an Isoparaffinen reichen Austrittsfluids entnimmt, um die zweite Stufe der Unter-Druck-Setzung RP2 des folgenden Adsorbers 3 in aufsteigendem Strom zu realisieren;
d) eine Sequenz 4, in welcher die Adsorptionsstufe (A/F) fortgesetzt wird, indem man über eine Leitung 4 das obere Ende des Adsorbers 2 mit dem Boden des folgenden Adsorbers 3, vorher in Phase der zweiten Unter-Druck-Setzung, verbindet, wobei der Adsorber 2 die dann mit n-Paraffinen gesättigte Charge über die Leitung 3 aufnimmt, und die Stoffübertragungszone sich gegen den Adsorber entsprechend 3, in Reihe geschaltet, verschiebt;
e) eine Sequenz 5, in der eine erste Druckentlastung (DP1) vorgenommen wird, indem man über den Boden über eine Leitung 5 den bei hohem Druck arbeitenden Adsorber 2 mit dem Adsorber 4, der seine Spülstufe bei einem geringeren Druck beendet hat und so ein Druckgleichgewicht in den Adsorbern 2 und 4 erhält, verbindet, wobei das Adsorbens im Adsorber 2 dann völlig bei Chargengleichgewicht gesättigt ist;
f) eine Sequenz 6, in welcher man eine zweite Druckentlastungsstufe (DP2) bei geschlossenem oberen Ende des Adsorbers 2 realisiert, wobei der Abstrom dieser Sequenz am Boden des Adsorbers 2 über eine Leitung 6 abgezogen wird;
g) eine Sequenz 7, in der man eine erste Spülphase (S1) realisiert, indem man den Boden des vorhergehenden Adsorbers 1 mit dem Kopf des Adsorbers 2 über eine Leitung 8 in Reihe verbindet, indem man das Desorptionsmittel über eine Leitung 7 zum Kopf des Adsorbers 1 bei Ende des Spülvorgangs S2 einführt, wobei der dort austretende Abstrom dann relativ arm an der adsorbierbaren Fraktion ist, und das über die Leitung 6 des Adsorbers 2 austretende Produkt im wesentlichen aus einer an n-Paraffinen reichen Fraktion besteht;
h) zwei Sequenzen 8 und 9, in welchen man die Hauptstufe der Spülung (STR) realisiert, indem man allein den Adsorber 2 über das Desorptionsmittel in absteigender Strömung über eine Leitung 9 einspeist;
i) eine Sequenz 10, in der man eine Stufe zur Beendigung des Spülens (S2) realisiert, indem man weiterhin den Adsorber 2 über die Leitung 9 mit Desorptionsmittel speist und über die Leitung 10 den Ausgang dieses Adsorbers 2 mit dem folgenden Adsorber 3 verbindet, der die Desorptionsphase (S1) einleitet;
j) eine Sequenz 11, in der man eine Unter-Drucksetzung (RP1) des niedrigen Drucks auf einen Zwischendruck realisiert, indem man die Adsorber 2 und 4 am Boden über eine Leitung 11 verbindet und
k) eine Sequenz 12, in der man eine zweite Unter-Druck-Setzung (RP2) bis zum Adsorptionsdruck vermittels eines Flusses oder Stroms realisiert, der über eine Leitung 12 zum Teil aus dem Abstrom des vorhergehenden Adsorbers 1 stammt, welcher in einer Adsorptionsstufe (ADS) arbeitet, wobei das Fluid im unteren Teil des Adsorbers 2 eingeführt wird, wobei als vereinbart gilt, dass:
- der Adsorber 3 seine Sequenz 1 beginnt, wenn der Adsorber 2 seine Sequenz 4 beginnt;
- der Adsorber 4 seine Sequenz 1 beginnt, wenn der Adsorber 2 seine Sequenz 7 beginnt und
- der Adsorber 1 seine Sequenz 1 beginnt, wenn der Adsorber 2 seine Sequenz 10 beginnt.

2. Verfahren nach Anspruch 1, dadurch gekennzeichnet, dass in den Sequenzen 7 bis 10 das Spülen mit Hilfe eines an Isoparaffinen reichen Gasstroms vorgenommen wird.

3. Verfahren nach Anspruch 2, dadurch gekennzeichnet, dass das Desorptionsmittel einen Anteil an n-Paraffinen zwischen 0 und 20 % enthält.

4. Verfahren nach einem der Ansprüche 1 bis 3, dadurch gekennzeichnet, dass das Adsorberbett gebildet wird durch ein Molekularsieb auf Zeolitbasis, das in der Lage ist, selektiv die n-Paraffine zu adsorbieren und über einen scheinbaren Porendurchmesser benachbart 5 Angström verfügt.

5. Verfahren nach Anspruch 4, dadurch gekennzeichnet, dass das Adsorberbett gebildet wird durch Zeolith 5a, Chabazit und Erionit.

6. Verfahren nach einem der Ansprüche 1 bis 5, dadurch gekennzeichnet, dass man bei Temperaturen von 100 bis 400 °C und bei einem Adsorptionsdruck von 5 bis 40 bar arbeitet, wobei der Adsorptionszyklus 2 bis 15 Minuten dauert.

7. Verfahren nach Anspruch 6, dadurch gekennzeichnet, dass man bei Temperaturen von 200 bis 300 °C und bei einem Adsorptionsdruck von 15 bis 25 bar arbeitet, wobei der Adsorptionszyklus 4 bis 6 Minuten dauert.

8. Verfahren nach einem der Ansprüche 1 bis 7, dadurch gekennzeichnet, dass in der Stufe g) die Spülphase bei einem Druck von weniger als 5 bar absolut durchgeführt wird.

9. Verfahren nach einem der Ansprüche 1 bis 7, dadurch gekennzeichnet, dass in der Stufe g) die Spülphase bei einem Druck von weniger als 3 bar absolut durchgeführt wird.

10. Verfahren nach einem der Ansprüche 1 bis 9, dadurch gekennzeichnet, dass die behandelte Charge aus einer Einheit zur Isomerisierung der C5/C6 stammt.

11. Verfahren nach Anspruch 10, dadurch gekennzeichnet, dass der Desorptionsabstrom des Adsorbers 2 zur Isomerisierungseinheit der C5/C6 rezykliert wird, wobei der Boden des Adsorbers 2 mit dem Rezyklierungskreis zur Isomerisierung verbunden und bei niedrigem Druck in den Sequenzen 6, 7, 8, 9 gehalten wird.
